# EUROPÄISCHE PATENTANMELDUNG

(11) **EP 4 659 770 A1**
(43) Veröffentlichungstag der Anmeldung: **10.12.2025**
(21) Anmeldenummer: 24179585.5
(22) Anmeldetag: 03.06.2024
(51) Int. Cl.: A61L 27/18, A61L 27/50, A61L 27/56

(54) **GEWEBEERSATZ MIT STÜTZSCHICHT, ABDICHTENDER POLYMERFOLIE UND VLIESSCHICHT**

(71) Anmelder: Fraunhofer-Gesellschaft zur Förderung der angewandten Forschung e.V., 80686 München (DE); NMI Naturwissenschaftliches und Medizinisches Institut an der Universität Tübingen, 72770 Reutlingen (DE)
(72) Erfinder: Meyer, Wolfdietrich, 42653 Solingen (DE); Bakhshi, Hadi, 14478 Potsdam (DE); Hartmann, Hanna, 72072 Tübingen (DE); Visser, Dmitri, 72070 Tübingen (DE); Schenke-Layland, Katja, 72827 Wannweil (DE)
(74) Vertreter: Abitz & Partner

(57) **Zusammenfassung**

Die Erfindung betrifft einen biokompatiblen Gewebeersatz für das Perikard, umfassend
- eine elastomere, und biokompatible Stützschicht
- eine gegenüber wässrigen Flüssigkeiten abdichtende Polymerfolie,
- eine Vliesschicht, wobei die Vliesschicht gesponnene, biokompatible Polymerfasern umfasst,
- wobei die abdichtende Polymerfolie zwischen der Vliesschicht und der Stützschicht angeordnet ist.

Ein derartiger Gewebeersatz ist aufgrund seiner mechanischen Stabilität besonders gut geeignet als Gewebeersatz zu dienen.

## Beschreibung

Der Ersatz von Gewebe im menschlichen oder tierischen Körper und die Rekonstruktion von Gewebe stellen eine große technische Herausforderung dar. Aufgrund der steigenden Zahl von Menschen mit Herzinsuffizienz besteht ein steigender Bedarf an Ersatz für kardiales Gewebe. Der Herzbeutel oder das Perikard ist ein Sack aus Bindegewebe, der zum Schutz das Herz umgibt, gleichzeitig aber aufgrund seiner Flexibilität dem Herzen auch genügend Freiraum für die Kontraktionswellen lässt, die das Blut durch den Körper pumpen. Als Ersatz für den Herzbeutel, zur Rekonstruktion von Gefäßen oder als Ersatz für Herzklappen wird heute üblicherweise Herzbeutel-Gewebe des Schweines oder des Rindes verwendet, das intensiven und aufwändigen Dezellularisierungsprozessen unterzogen wird, um eine Abstoßung des Herzbeutel-Gewebes bei der Implantation zu verringern bzw. zu unterbinden. Diese Dezellularisierungsprozesse sind aufwendig und teuer und führen häufig zu starken Qualitätsunterschieden der Gewebe, die den Einsatz als Gewebeersatz bzw. als Implantat verkomplizieren und auch in Abhängigkeit vom Spendertier variieren können. Dies erschwert die Bereitstellung einer großen Anzahl von Implantaten für die Herzchirurgie. Die Bereitstellung von Herzbeutel-Geweben des Schweines oder Rindes führt auch zu ethischen und religiösen Problemen. Ähnliche Probleme treten auch auf, wenn ein Gewebeersatz für zum Beispiel Gefäße wie Blutgefäße zur Verfügung gestellt werden soll. Blutgefäße dehnen sich ebenso wie das Perikard elastisch aus und ziehen sich wieder zusammen. Daher wird das Perikard auch häufig als Standard für andere Implantate, beispielsweise Herzklappen verwendet.

Aus der Veröffentlichung Visser et al.: "Green Chemistry for Biomimetic Materials: Synthesis of High-Molecular-Weight Polycarbonate-Based Nonisocyanate Polyurethanes", ACS Omega 2022, 39772, ist das Elektrospinnen von thermoplastischen Polyurethanen zu Geweben bekannt, deren Einsatz als Gewebeersatz für das Perikard in Betracht gezogen wird. Die Gewebe erlauben die Adhäsion von Zellen, sind aber aufgrund ihrer mechanischen Eigenschaften nicht für den Ersatz des Perikards geeignet.

Die Aufgabe der vorliegenden Erfindung besteht darin, einen Gewebeersatz bereitzustellen, der bezüglich der oben genannten Nachteile verbessert ist. Gegenstand weiterer Patentansprüche ist die Verwendung des Gewebeersatzes, sowie ein Verfahren zur Herstellung des Gewebeersatzes.

Die Erfindung stellt einen biokompatiblen Gewebeersatz zur Verfügung. Der Gewebeersatz umfasst.
- eine elastomere und biokompatible Stützschicht und
- eine gegenüber wässrigen Flüssigkeiten abdichtende Polymerfolie, eine Vliesschicht, wobei die Vliesschicht gesponnene, biokompatible Polymerfasern umfasst,
- wobei die abdichtende Polymerfolie zwischen der Vliesschicht und der Stützschicht angeordnet ist.

Die Biokompatibilität des gesamten Gewebeersatzes kann dadurch erreicht werden, dass alle für den Gewebeersatz verwendeten Materialien biokompatibel im Sinne der Norm EN ISO 10993-5 sind. Die Biokompatibilität der Stützschicht und des gesamten Gewebeersatzes kann gemäß der Norm EN ISO 10993-5 bestimmt werden.

Unter "elastomeren Polymeren" im Sinne der vorliegenden Erfindung werden formfeste, aber elastisch verformbare Kunststoffe verstanden. Bei Zug- und Druckbelastung können sich die elastomeren Polymere elastisch verformen, finden aber bei Beendigung der Zug- und Druckbelastung wieder in ihre ursprüngliche, unverformte Gestalt zurück.

Eine "Vliesschicht" im Sinne der vorliegenden Erfindung ist eine Schicht, die aus Fasern gebildet wird, die ohne eine Vorzugsrichtung oder eine periodische Verknüpfung miteinander verschlungen sind und daher eine Wirrlagen-Schicht bilden.

Die Stützschicht ist dabei ausgelegt die Vliesschicht und die abdichtende Polymerfolie zu tragen und erhöht die mechanische Stabilität der auf der Stützschicht angeordneten Vliesschicht. Aufgrund der elastomeren Eigenschaften der Stützschicht ist der Gewebeersatz für das Perikard oder für Gefäße, wie beispielsweise Blutgefäße geeignet und ermöglicht eine Aufnahme des Herzens als Herzbeutel, wobei der Gewebeersatz aufgrund seiner Flexibilität auch die Kompression des Herzens während der Kontraktionswellen erlaubt.

Die elastomere, biokompatible Stützschicht kann eine Zugfestigkeit aufweisen, die größer ist als die Zugfestigkeit der Vliesschicht. Dies ermöglicht es besonders einfach, dass die Stützschicht der Vliesschicht eine größere mechanische Stabilität verleiht. Die Zugfestigkeit der Stützschicht und die Zugfestigkeit der Vliesschicht können insbesondere gemäß der Norm DIN EN ISO 527 bestimmt werden. Insbesondere kann die Zugfestigkeit der elastomeren, biokompatiblen Stützschicht 58 MPa bis 65 MPa, insbesondere 62 MPa betragen. Die Zugfestigkeit der Vliesschicht kann sich im Bereich von 50 MPa bis 56 MPa bewegen.

Die Vliesschicht, die die gesponnenen und biokompatiblen Polymerfasern umfasst, kann die extrazelluläre Matrix im menschlichen Körper imitieren. Die Vliesschicht ist dafür ausgelegt, zur Adhäsion und Proliferation von Zellen zu dienen. Dies kann insbesondere eine Besiedelung der Vliesschicht des Gewebeersatzes nach der Implantation im Körper mit Zellen ermöglichen. Die Vliesschicht kann insbesondere dazu ausgelegt sein, die Adhäsion und Proliferation von Fibroblasten und Epithelzellen zu ermöglichen. Die Vliesschicht kann aus den gesponnenen, biokompatiblen Polymerfasern gefertigt sein. Bei einer Verwendung des biokompatiblen Gewebeersatzes für das Perikard kann die Vliesschicht insbesondere in Kontakt mit dem Perikard des Patienten stehen bzw. dem Herzen des Patienten gegenüber liegen. In diesem Fall zeigt die elastomere und biokompatible Stützschicht dann nach außen, vom Perikard bzw. vom Herzen des Patienten weg. Möglich sind auch Verwendungen des biokompatiblen Gewebeersatzes, bei denen die Stützschicht im Kontakt mit dem Körpergewebe eines Patienten steht.

Die gegenüber wässrigen Flüssigkeiten abdichtende Polymerfolie kann insbesondere gegenüber biologischen Flüssigkeiten, insbesondere Körperflüssigkeiten abdichten. Die gegenüber wässrigen Flüssigkeiten abdichtende Polymerfolie kann insbesondere Zellschichten des zu ersetzenden Gewebes imitieren. Beispielsweise kann die gegenüber wässrigen Flüssigkeiten abdichtende Polymerfolie das Epikard imitieren, falls der Gewebeersatz für das Perikard eingesetzt wird. Die gegenüber wässrigen Flüssigkeiten abdichtende Polymerfolie kann auch das Endothel imitieren, falls der Gewebeersatz als Ersatz für Blutgefäße eingesetzt werden soll.

Die gegenüber wässrigen Flüssigkeiten abdichtende Polymerfolie ist zwischen der Stützschicht und der Vliesschicht angeordnet. Die gegenüber wässrigen Flüssigkeiten abdichtende Polymerfolie ist insbesondere dazu ausgelegt, den Transport von wässrigen Flüssigkeiten zwischen der Stützschicht und der Vliesschicht zu verhindern.

Somit ist die gegenüber wässrigen Flüssigkeiten abdichtende Polymerfolie auf der elastomeren und biokompatiblen Stützschicht angeordnet. Die Vliesschicht ist auf der abdichtenden Polymerfolie angeordnet.

Die gegenüber wässrigen Flüssigkeiten abdichtende Polymerfolie kann einen Kunststoff umfassen oder aus diesem gefertigt sein. Als Kunststoffe kommen beispielsweise Polyurethan, Silikone, Polyisopren, Polyolefine, Polyamide, Acrylate oder Kombinationen davon in Betracht. Besonders bevorzugt umfasst die abdichtende Polymerfolie Polyurethan oder ist die abdichtende Polymerfolie aus Polyurethan gefertigt.

Um gegenüber wässrigen Flüssigkeiten, insbesondere gegenüber biologischen, wässrigen Flüssigkeiten abzudichten weist die abdichtende Polymerfolie bevorzugt eine Oberflächen-Hydrophobizität auf. Insbesondere weist die Oberfläche der abdichtenden Polymerfolie einen Kontaktwinkel von zumindest 90° gegenüber Wasser auf und ist damit wasserabweisend. Eine derartige wasserabweisende Oberfläche kann durch Verwendung eines intrinsisch hydrophoben Polymers, wie beispielsweise Polyurethan Silikon, Polyisopren, Polyolefine, Polyamide, Acrylaten oder Kombinationen davon erzielt werden. Bevorzugt umfasst die abdichtende Polymerfolie Polyurethane. Weiterhin kann das intrinsische hydrophobe Polymer eine hohe Dichte von zumindest 1,05 g/cm³ aufweisen. Insbesondere kann die abdichtende Polymerfolie eine Wasseraufnahme von weniger als 0,1 % aufweisen. Die abdichtende Polymerfolie kann weiterhin eine Bruchdehnung von 400 % aufweisen. Die abdichtende Polymerfolie kann auch ein Zugmodul bei 100 % Dehnung von 2,8 MPa aufweisen. Weiterhin kann die abdichtende Polymerfolie einen Biegemodus von 10,3 MPa aufweisen.

Die abdichtende Polymerfolie kann eine Dichte von 1,05 g/cm³ bis 1,3 g/cm³, bevorzugt eine Dichte von 1,19 g/cm³ aufweisen. Die Dicke der abdichtende Polymerfolie kann 50 µm bis 100 µm betragen.

Die abdichtende Polymerfolie kann bevorzugt mittels Extrusion, Heißpressen oder Lösungsgießen hergestellt werden. Insbesondere kann die abdichtende Folie mit der Stützschicht mittels eines Laminier-Vorgangs oder mittels Verklebung mit Lösungsmitteln wie THF und/oder DMF verbunden werden. Die abdichtende Folie kann insbesondere eine Shore-Härte von 50A bis 78A, bevorzugt von 60A bis 78A, weiter bevorzugt von 77A aufweisen.

Der Gewebeersatz für das Perikard kann ein zellfreier Gewebeersatz sein. Dies bedeutet, dass der Gewebeersatz weder menschliche Zellen noch Zellen eines möglichen Spendertiers enthält. Dies ermöglicht eine Implantation des Gewebeersatzes ohne Abstoßungsreaktionen des Körpers.

Die Stützschicht kann als Körper, insbesondere als Hohlkörper ausgeformt sein. Die Stützschicht kann eine Form aufweisen, die dem zu ersetzenden Körpergewebe nachempfunden ist. Beispielsweise kann die Stützschicht als Beutel ausgeformt sein, wenn der Gewebeersatz als Ersatz oder als zusätzliche Stütze für den Herzbeutel verwendet wird. Die Stützschicht kann auch als röhrenförmiger Körper ausgeformt sein. Dies kann beispielsweise dann vorteilhaft sein, wenn der Gewebeersatz als Ersatz für Gefäße, beispielsweise Blutgefäße zum Einsatz kommt.

Die auf der Stützschicht angeordnete Vliesschicht und auch die abdichtende Polymerfolie kann der Formgebung der Stützschicht folgen. Die Stützschicht kann insbesondere als formgebendes Stützgerüst innerhalb des Gewebeersatzes dienen. Die körperliche Form des Gewebeersatzes kann der Formgebung der Stützschicht folgen.

Die Stützschicht kann weiterhin auch als ebene Fläche ausgeformt sein. Dies kann dann von Vorteil sein, wenn der Gewebeersatz beispielsweise als Wundauflage oder Pflaster verwendet werden soll. In diesem Fall kann die Vliesschicht in Richtung der Wunde orientiert werden, während die Stützschicht nach außen weist. Die Stützschicht kann dann insbesondere auch Barrierefunktionen erfüllen und beispielsweise verhindern, dass Schmutz oder Pathogene von außen in die Wunde eintreten können.

Die Stützschicht kann weiterhin eine Härte aufweisen, die größer ist als die Härte der abdichtenden Polymerfolie. Insbesondere kann die Stützschicht eine Shore-Härte von 80A bis 90A, bevorzugt von 82A bis 88A aufweisen. Die Shore-Härte kann mittels der Norm DIN ISO 7619-1 bestimmt werden.

Die Stützschicht kann einen ersten Flächenbereich und einen vom ersten Flächenbereich beabstandeten zweiten Flächenbereich umfassen. Zumindest ein, sich schlängelnder Verbindungssteg ist vorhanden, der den ersten Flächenbereich mit dem zweiten Flächenbereich verbindet. Der sich schlängelnde Verbindungssteg zwischen dem ersten Flächenbereich und dem zweiten Flächenbereich ist besonders gut dazu ausgelegt, eine mechanische Eigenschaft der Stützschicht zu bedingen. Insbesondere ist der sich schlängelnde Verbindungssteg, der den ersten Flächenbereich mit dem zweiten Flächenbereich verbindet, dazu ausgelegt, der Stützschicht eine nicht-lineare Dehnung in einem Bereich der Dehnung von 0 % bis 20 % zu verleihen.

Insbesondere kann es vorteilhaft sein, wenn eine Vielzahl von nebeneinander sich schlängelnden Verbindungsstegen den ersten Flächenbereich mit dem zweiten Flächenbereich verbinden. Beispielsweise können bis zu 40 Verbindungsstege vorhanden sein, die schlängelnd den ersten Flächenbereich mit dem zweiten Flächenbereich verbinden. Insbesondere können zumindest drei, bevorzugt zumindest fünf, weiter bevorzugt zumindest zehn Verbindungsstege vorhanden sein. Eine zunehmende Anzahl von Verbindungsstegen verbessert die Stützfunktion der Stützschicht, erlaubt aber weiterhin eine nicht-lineare Dehnung in einem Bereich der Dehnung von 0 % bis 20 %. Insbesondere können die sich schlängelnden Verbindungsstege zwischen dem ersten Flächenbereich und dem zweiten Flächenbereich eine Breite von 400 µm bis 3 mm aufweisen. Der Abstand zwischen benachbarten, sich schlängelnden Verbindungsstegen kann zwischen 1,2 mm bis 9 mm betragen.

Der erste und der zweite Flächenbereich dienen insbesondere zu einer zuverlässigen Anbindung der Stützschicht an die abdichtende Polymerfolie. Der erste und der zweite Flächenbereich dienen auch dazu, die Vielzahl der sich nebeneinander schlängelnden Verbindungsstege räumlich zuverlässig zueinander auszurichten.

Die Stützschicht kann weiterhin auch eine sich wiederholende Anordnung von Wiederholeinheiten umfassen oder daraus aufgebaut sein. Die Wiederholeinheiten können von durch Trennwände voneinander abgetrennte Hohlräume umfassen oder daraus ausgebaut sein.

Die Hohlräume können eine ovale Form oder eine polygonale Form aufweisen. Die mittels der Trennwände voneinander abgetrennten Hohlräume können eine trigonale, tetragonale, pentagonale, hexagonale oder heptagonale Form aufweisen.

Die sich wiederholende Anordnung von Wiederholeinheiten kann insbesondere eine mechanische Eigenschaft, insbesondere eine nicht-lineare Dehnung in einem Bereich einer Dehnung von 0% bis 20% der Stützschicht bedingen, beispielsweise eine erhöhte Zugfestigkeit der Stützschicht.

Bevorzugt weisen die Hohlräume der Wiederholeinheiten eine hexagonale Form auf. Die Stützschicht kann insbesondere eine Wabenstruktur mit hexagonalen Hohlräumen als Wiederholeinheiten aufweisen. Eine derartige Stützschicht ist besonders gut dazu geeignet, eine Zugfestigkeit aufzuweisen, die der des Perikard-Gewebes entspricht.

Die Stützschicht kann insbesondere als Metamaterial ausgeformt sein. Unter Metamaterialien im Sinne der vorliegenden Erfindung werden makroskopische Werkstoffe mit einem künstlichen, dreidimensionalen periodischen Aufbau verstanden. Der 3-dimensionale periodische Aufbau bedingt eine mechanische Eigenschaft der Stützschicht, die nicht auf das Material der Stützschicht alleine zurückzuführen ist. Der künstliche, dreidimensionale periodische Aufbau kann im Fall der vorliegenden Erfindung aus der sich wiederholenden Anordnung von Wiederholeinheiten, insbesondere den Hohlräumen aufgebaut sein. Die künstliche, dreidimensionale periodische Architektur kann auch aus der Vielzahl sich nebeneinander schlängelnder Verbindungsstege der Stützschicht, wie oben beschrieben, aufgebaut sein.

Die sich wiederholende Anordnung von Wiederholeinheiten der Stützschicht kann beispielsweise mittels 3-D Druck, beispielsweise Stereolithographie, mittels Laserschnitt, mittels Fused Deposition Modeling, oder Tauchbeschichtung erzeugt werden.

Die Stützschicht kann im Spannungs-Dehnungs-Diagramm eine nicht-lineare Dehnung in einem Bereich einer Dehnung zwischen 0 % und 20 % aufweisen, bevorzugt kann die Stützschicht eine Zugfestigkeit von 0,003 N/mm² bis 0,1 N/mm², bevorzugt von 0.00374 N/mm² bis 0.09138 N/mm² bei einer Dehnung zwischen 0% und 10% aufweisen. Die Stützschicht kann weiterhin eine Zugfestigkeit von 0,1 N/mm² bis 0,7 N/mm², bevorzugt von 0.09138 N/mm² bis 0.69029 N/mm² bei einer Dehnung zwischen 10% und 20% aufweisen.

Die Zugfestigkeit der Stützschicht kann dabei größer sein als die Zugfestigkeit der Vliesschicht. Dies kann dazu führen, dass der gesamte Gewebeersatz die Zugfestigkeit der Stützschicht aufweist.

Eine derartige Zugfestigkeit im Spannung-Dehnung-Diagramm entspricht etwa der Zugfestigkeit des Perikard-Bindegewebes. Insbesondere kann es bei Ausübung eines geringen Zuges mit einer Kraft bis 0,5 MPa (0,5 N/mm²) zu einer großen Dehnung des Gewebeersatzes im Bereich bis 10-15 % kommen. Die Prozentangaben für die Dehnung beziehen sich auf die ursprüngliche Größe des Gewebes vor Ausübung der Kraft. Dies ermöglicht es besonders einfach den Gewebeersatz für das Perikard einzusetzen. Die Zugfestigkeit der Stützschicht kann durch Veränderung der Dicke der Stützschicht reguliert werden. Insbesondere kann die Dicke der Stützschicht in einem Bereich von 50 µm bis 300 µm angepasst werden, wobei die Zugfestigkeit mit zunehmender Dicke erhöht wird.

Das elastomere Polymer der Stützschicht kann ausgewählt sein aus einer Gruppe bestehend aus: Polyurethanelastomeren, Silikonelastomeren, Polyisopren, Olefinelastomeren, Ethylen-Propylen-Dien-Kautschuken, Ethylen-Propylen-Kautschuke, Styrol-Blockcopolymere und Polyetherblock-Amiden, Acrylat-Elastomere und deren Copolymere und Kombinationen der genannten Polymere. Bevorzugt ist das elastomere Polymer der Stützschicht ein Copolymer umfassend Urethan-Wiederholeinheiten und Acrylat-Wiederholeinheiten, bevorzugt Polyurethan(meth)acrylat. Diese Polyurethan(meth)acrylate können insbesondere durch 3D-Drucken, beispielsweise Stereolithographie gebildet werden.

Beispielsweise kann eine Mischung aus einem Urethandiacrylat als Vernetzer und einem monofunktionellen Urethanacrylat als Reaktivverdünner zusammen mit einem Fotoinitiator verwendet werden.

Bei dem Urethandiacrylat kann es sich beispielsweise um folgende allgemeine Verbindung handeln: 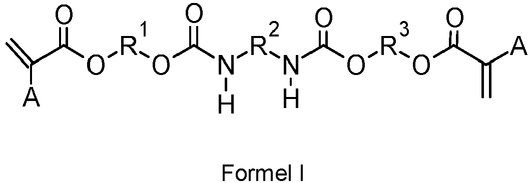
wobei die Gruppe A für H oder CH₃ steht,
die Gruppen R¹ und R³ unabhängig voneinander für eine zweiwertige verzweigte oder unverzweigte C₂ bis C₆-Kohlenwasserstoff-Gruppe stehen, und
die Gruppe R² eine zweiwertige verzweigte oder unverzweigte C₄ bis C₁₅-Kohlenwasserstoff-Gruppe oder eine zweiwertige Kohlenwasserstoff-Aryl-Gruppe ist.

Bei dem Urethandiacrylat kann es sich insbesondere um die Verbindung 7,7,9-Trimethyl-4,13-dioxo-3,14-dioxa-5,12-diazahexadecan-1,16-diol dimethacrylat: 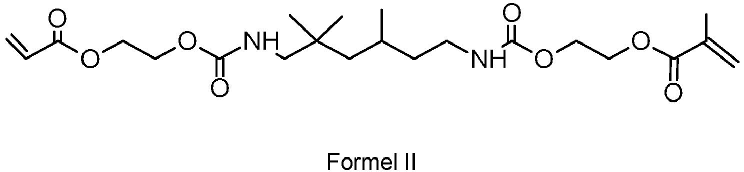 handeln.

Bei dem monofunktionellen Urethanacrylat kann es sich beispielsweise um folgende allgemeine Verbindung handeln: 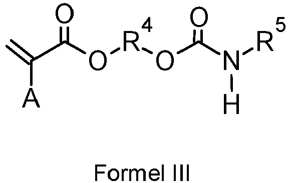
wobei die Gruppe A für H oder CH₃ steht, und
die Gruppen R⁴ und R⁵ unabhängig voneinander für eine verzweigte oder unverzweigte C₂ bis C,s-alkyl Kohlenwasserstoff-Gruppe stehen, wobei R⁴ eine zweiwertige Gruppe und R⁵ eine einwertige Gruppe darstellen.

Das Urethanacrylat kann insbesondere 2-[[(butylamino)carbonyl]oxy]ethyl 2-propenoat: 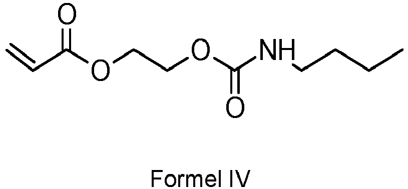 sein.

Als Fotoinitiator können beispielsweise Verbindungen verwendet werden, die unter UV-Licht zu Radikalen zerfallen und damit eine radikalische Fotopolymerisation der Urethanacrylate auslösen können. Als Fotoinitiator kann beispielsweise Ethyl(2,4,6-trimethylbenzoyl)phenylphosphinat verwendet werden.

Die Stützschicht des Gewebeersatzes für das Perikard kann daher ein Polyurethan mit folgenden Wiederholeinheiten umfassen, die durch Polymerisation des Urethandiacrylats der allgemeinen Formel I erhalten werden: 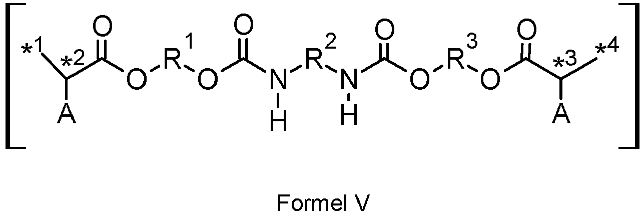
Wobei die allgemeinen Gruppen A, R¹, R² und R³ die oben bezeichneten Bedeutungen für die allgemeine Formel I aufweisen,
   und
wobei die mit *¹ und *² beziehungsweise die mit *³ und *⁴ bezeichneten Positionen jeweils alternative Positionen für die Anbindung an weitere Wiederholeinheiten oder an eine Endgruppe des Polyurethans bezeichnen.

Die Stützschicht des Gewebeersatzes für das Perikard kann weiterhin ein Polyurethan mit der folgenden allgemeinen Endgruppe umfassen, die durch den Einbau des monofunktionellen Urethanacrylats der allgemeinen Formel III in die Polyurethane erhalten werden kann: 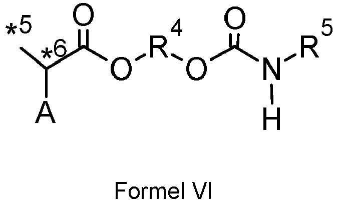
wobei die allgemeinen Gruppen A, R⁴ und R⁵ die für die allgemeine Formel III beschriebenen Bedeutungen aufweisen, und
wobei die mit *⁵ und *⁶ bezeichneten Positionen alternative Positionen für die Anbindung der Endgruppe an den Rest des Polyurethans bezeichnet.

Die Dicke der Stützschicht des Gewebeersatzes kann von 50 µm bis 300 µm, bevorzugt von 80 µm bis 150 µm betragen.

Eine derartige Schichtdicke der Stützschicht ist besonders gut dazu geeignet, die Vliesschicht zu tragen und abzustützen.

Die Polymere der gesponnenen Polymerfasern der Vliesschicht können ausgewählt sein aus: Polyurethan, Polymilchsäure (PLA), Polycaprolacton, Poly(lactid-co-glycolid), Polyethylenoxid, Polyvinylalkohol und Strukturproteinen, Co-Polymeren davon und Kombinationen der genannten Polymere. Unter Strukturproteinen werden dabei Proteine verstanden, die in Geweben und Zellen von Lebewesen als Gerüst-Proteine dienen. Die Strukturproteine können insbesondere ausgewählt sein aus einer Gruppe bestehend aus: Kollagen, Keratin, und Elastin.

Besonders bevorzugt können die Polymere der gesponnenen Polymerfasern der Vliesschicht Isocyanat-freies Polyurethan umfassen, oder aus diesen gefertigt sein. Aufgrund der Umwelt- und Gesundheitsschädlichkeit der Isocyanate sollten diese nach Möglichkeit vermieden werden. Die Synthese von Isocyanat-freien Polyurethanen kann durch die Polykondensation von Dicarbamaten und Diolen erfolgen. Dicarbamate können durch die Kondensation von Diaminen mit Dialkylcarbonaten gebildet werden.

Insbesondere können die Isocyanat-freien Polyurethane der Vliesschicht mittels folgender allgemeiner Polykondensationsreaktion aus den Dicarbamaten der allgemeinen Formel VII und den Diolen der allgemeinen Formel VIII erhalten werden: 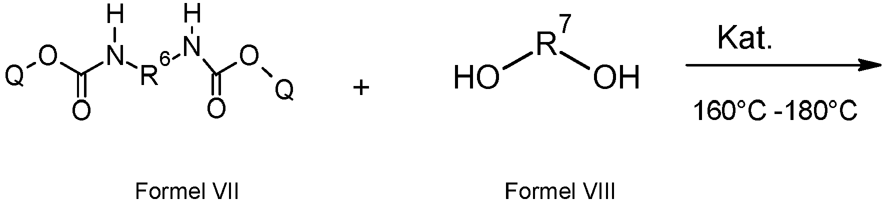 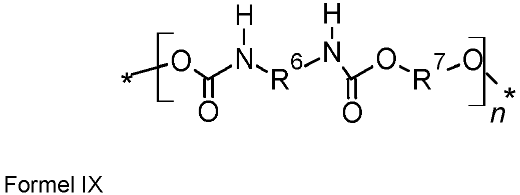
wobei die allgemeine Gruppe Q verzweigte oder unverzweigte C₁ bis C₆-Alkylgruppen bezeichnet, bevorzugt eine Methyl- oder Ethyl-Gruppe,
wobei die allgemeine Gruppe R⁶ zweiwertige verzweigte oder unverzweigte C₂ bis C₁₂-Kohlenwasserstoff-Gruppen, bevorzugt eine zweiwertige Hexyl-Gruppe, bezeichnet,
wobei die Zahl n die Anzahl der Wiederholeinheiten im Polyurethan der Formel IX beschreibt, und wobei gilt 4 ≤ n ≤ 36,
wobei die allgemeine Gruppe R⁷ ausgewählt ist aus einer Gruppe bestehend aus: zweiwertige verzweigte oder unverzweigte C₂ bis C₁₂-Kohlenwasserstoff-Gruppen, wobei die Kohlenwasserstoff-Gruppen auch eine zweiwertige Carbonat-Gruppe aufweisen können, und Proteinen, insbesondere den oben genannten Strukturproteinen,
wobei die mit * bezeichnete Positionen in der Formel IX die Anbindung an weitere Wiederholeinheiten innerhalb der Polyurethane bezeichnet. Als Katalysator (Kat.) können beispielsweise Titanat-Katalysatoren, wie Tetrabutyl-Titanat (TBT) verwendet werden

Die allgemeine Gruppe R⁷ kann insbesondere die folgende allgemeine Gruppe R⁸ sein: 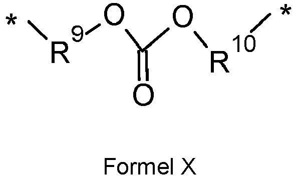
wobei die mit * bezeichnete Positionen in der Formel X die Anbindung an die beiden terminalen O- Atome der allgemeinen Gruppe R⁷ bezeichnen, und
wobei die allgemeinen Gruppen R⁹ und R¹⁰ unabhängig voneinander zweiwertige verzweigte oder unverzweigte Kohlenwasserstoff-Gruppen bezeichnen, mit der Maßgabe, dass beide allgemeinen Gruppen R⁹ und R¹⁰ zusammen mindestens 2 Kohlenstoff-Atome und höchstens 12 Kohlenstoff-Atome aufweisen.

Da die Strukturproteine, beispielsweise Kollagen, Elastin oder Keratin ebenfalls OH-Gruppen aufweisen können diese ebenfalls über die oben genannte Polykondensation als Wiederholeinheiten in Polyurethan-Copolymere eingebaut werden.

Weiterhin ist es auch möglich Vliesschichten aus elastomeren, biokompatiblen Polymeren, beispielsweise Kunststoffen herzustellen und diese Vliesschichten anschließend mit den Strukturproteinen zu funktionalisieren. Dies kann beispielsweise dadurch geschehen, dass die Vliesschichten mit Lösungen der Strukturproteine, beispielsweise Kollagenlösungen inkubiert werden, wobei sich die Strukturproteine auf der Oberfläche der Fasern abscheiden. Beispielsweise kann Kollagen aus einer tierischen Quelle, beispielsweise aus Rattenschwänzen isoliert werden. Ein Lyophilisat des Kollagens kann in leicht saurer Lösung (0,1 M Essigsäure) gelöst und eine bereits gesponnene Vliesschicht in die Lösung für einen Zeitraum von 1 Stunde bis 24 Stunden bei einer Temperatur von 20 °C bis 40 °C, bevorzugt 37 °C, eingelegt werden. Anschließend kann die Vliesschicht getrocknet werden, wobei die mit Kollagen funktionalisierte Vliesschicht gebildet wird.

Die Isocyanat-freien Polyurethane oder Polyurethan-Copolymere der Vliesschicht des Gewebeersatzes können daher Polyurethane der folgenden allgemeinen Formel umfassen, oder aus diesen gefertigt sein: 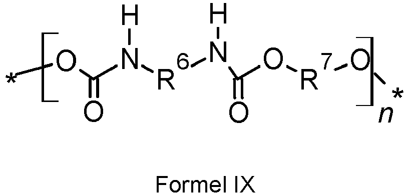 wobei der Anteil an Isocyanat 0 ppm ist. Bevorzugt kann die allgemeine Gruppe R⁷ die allgemeine Gruppe R⁸ der Formel X oder die oben genannten Strukturproteine sein.

Die gesponnenen Polymerfasern der Vliesschicht können einen durchschnittlichen Durchmesser (D₅₀) von 50 nm bis 6000 nm, bevorzugt einen durchschnittlichen Durchmesser von 50 nm bis 3000 nm aufweisen. Der Durchmesser der gesponnenen Polymerfasern kann mittels Rasterelektronenmikroskopie mit der Software "MATLAB" unter Verwendung des Algorithmus "Simpoly" bestimmt werden (Murphy, R.; Turcott, A.; Banuelos, L.; Dowey, E.; Goodwin, B.; Cardinal, K. O. SIMPoly: A matlab-based image analysis tool to measure electrospun polymer scaffold fiber diameter. Tissue Eng., Part C 2020, 26, 628-636).

Der durchschnittliche Durchmesser von gesponnenen natürlichen Strukturproteinen, wie beispielsweise Kollagen, kann bevorzugt zwischen 50 nm bis 300 nm betragen. Insbesondere können 67 % der Fasern in der Vliesschicht Strukturproteine mit einem Durchmesser von 80 nm bis 150 nm aufweisen. Der durchschnittliche Durchmesser von gesponnenen biokompatiblen synthetischen Polymerfasern kann bevorzugt zwischen 300 nm und 3 µm betragen. Insbesondere können 67 % der Fasern in der Vliesschicht synthetische Polymerfasern, wie Polyurethan mit einem Durchmesser von 300 nm bis 3 µm aufweisen.

Die Vliesschicht des Gewebeersatzes kann Zwischenräume mit einer durchschnittlichen Porengröße von mehr als 20 µm aufweisen. Eine derartige Größe der Zwischenräume ist dann besonders bevorzugt, wenn Körperzellen des Empfängers des Gewebeersatzes in die Vliesschicht einwandern sollen, damit der Gewebeersatz mit dem umgebenden Gewebe verwachsen soll. Die Verwachsung mit dem umgebenden Gewebe ist vor allem dann von Vorteil, wenn die Implantation des Gewebeersatzes als einmaliger Eingriff geplant ist, nach dem der Gewebeersatz integraler Bestandteil des Gewebes wird, oder wenn ein langsamer Abbau des Gewebeersatzes im Körper des Empfängers vorgesehen ist. Bei einer Porengröße von weniger als 20 µm der Zwischenräume wandern Körperzellen des Empfängers in der Regel nicht in die Vliesschicht ein, sondern bilden eine Zellschicht auf der Vliesschicht aus. Die Porengröße der Zwischenräume kann beispielsweise dadurch bestimmt werden, dass die Vliesschicht mit einer Gold-Palladium-Schicht mittels herkömmlicher Methoden beschichtet und anschließend die Porengröße der Zwischenräume mittels Rasterelektronenmikroskopie und numerischer Bildauswertung analysiert wird. Zur Bestimmung von Porengrößen bei Poren mit unterschiedlicher geometrischer Ausformung wird der Umfang einer Anzahl von Poren, zumindest 5 Poren, bevorzugt 10 Poren mittels eines Bildauswertungsprogramms, wie beispielsweise ImageJ bestimmt und deren Mittelwert berechnet. Der theoretische Durchmesser und folglich die Porengröße können auf Basis der Annahme kreisförmiger Porenform ermittelt werden.. Die Bestimmung des Porendurchmessers in elektrogesponnenen Vliesschichten wird auch in der Publikation Stadelmann, K., et al. (2022): "Development of a bi-layered cryogenic electrospun polylactic acid scaffold to study calcific aortic valve disease in a 3D co-culture model" Acta Biomaterialia, 140, 364-378, beschrieben auf die hiermit vollinhaltlich Bezug genommen wird.

Die Vliesschicht kann Polyurethan mit einem Molekulargewicht von wenigstens 14.000 g/mol, bevorzugt Polyurethan mit einem Molekulargewicht von wenigstens 18.000 g/mol, weiter bevorzugt Polyurethan mit einem Molekulargewicht von 18.000 g/mol bis 60.000 g/mol umfassen, oder daraus gefertigt sein. Polyurethane mit einem derartigen Molekulargewicht sind besonders dazu geeignet in der Vliesschicht die Anbindung von Zellen nach der Implantation zu ermöglichen. Die Molekulargewichte der Polyurethane können beispielsweise mittels Gelpermeations-Chromatographie bestimmt werden.

Die gesponnenen, Polymerfasern der Vliesschicht können ineinander verschlungene Wirrfasern sein. Die Wirrfasern weisen dabei keine Vorzugsrichtung in Bezug auf ihre Orientierung auf, sondern sind beliebig ineinander verschlungen. Die Polymerfasern sind bevorzugt vereinzelte, nicht miteinander verschmolzene Polymerfasern.

Bevorzugt ist die Vliesschicht direkt auf der abdichtenden Polymerfolie angeordnet. Die abdichtende Polymerfolie kann direkt auf der Stützschicht angeordnet sein. Dies ermöglicht eine besonders effektive Abstützung der Vliesschicht durch die Stützschicht. Bevorzugt kann die Vliesschicht direkt auf der Stützschicht abgeschieden sein. Dies kann beispielsweise durch Elektrospinnen oder Schmelzspinnen durchgeführt werden. Möglich ist auch mittels Klebens oder Fügens die Vliesschicht direkt mit der abdichtenden Polymerfolie zu verbinden.

Besonders bevorzugt ist der biokompatible Gewebeersatz somit als Gewebeersatz aus drei Schichten gefertigt. Insbesondere ist die Vliesschicht auf der abdichtenden Polymerfolie angeordnet, wobei die abdichtende Polymerfolie wiederum auf der Stützschicht angeordnet ist.

Der Gewebeersatz kann bevorzugt aus der Stützschicht, der abdichtenden Polymerfolie und der Vliesschicht bestehen. Ein derartiger dreischichtiger Gewebeersatz ist besonders einfach und kostengünstig herzustellen.

Der Gewebeersatz der vorliegenden Erfindung kann insbesondere als Implantat oder als Wundauflage verwendet werden. Insbesondere kann der Gewebeersatz als Implantat für das Perikard, als Gefäß-Implantat, insbesondere als Blutgefäß-Implantat oder als Herzklappe verwendet werden.

In Abhängigkeit von der Anwendung des Gewebeersatzes kann die Stützschicht als Gerüst für die Vliesschicht unterschiedlich geformt werden. Die Stützschicht kann insbesondere beutelförmig als Hohlkörper ausgeformt sein, wenn der Gewebeersatz als Ersatz oder zur Abstützung für das Perikard konzipiert ist. Die Stützschicht kann auch röhrenförmig ausgeformt sein, falls der Gewebeersatz als Gefäß-Implantat verwendet werden soll. Möglich ist auch eine flächige, ebene Ausformung der Stützschicht, falls der Gewebeersatz als Wundauflage oder als Pflaster verwendet wird.

Gegenstand der vorliegenden Erfindung ist auch ein Verfahren zur Herstellung eines Gewebeersatzes, wie oben beschrieben, umfassend die Verfahrensschritte:
A) Bereitstellen der elastomeren, und biokompatiblen Stützschicht, und der abdichtenden Polymerfolie,
B) Anordnen der abdichtenden Polymerfolie auf der Stützschicht, und
C) Erzeugen oder Anordnen der Vliesschicht auf der abdichtenden Polymerfolie.

Im Verfahrensschritt A) kann die Stützschicht mittels 3D Druck, bevorzugt mittels Stereolithographie bereitgestellt werden, wobei das Photopolymer durch Bestrahlung mit einem Laser ausgehärtet werden kann. Die Wellenlänge des Lasers kann 365 nm betragen. Weiterhin kann die Stützschicht im Verfahrensschritt A) auch mittels Fused Deposition Modeling oder Tauchbeschichtung bereitgestellt werden. Bei Fused Deposition Modeling wird mittels 3-D-Drucks ein Werkstück schichtweise aus einem schmelzfähigen Kunststoff aufgebaut.

Insbesondere kann im Verfahrensschritt A) die Stützschicht durch 3D Druck aus UrethanAcrylaten erzeugt werden. Diese Urethan-Acrylate erlauben die Herstellung einer biokompatiblen, elastomeren Stützschicht durch Bestrahlung der Urethan-Acrylate, wobei es zu einer Fotopolymerisation durch Polymerisation über die funktionellen Acrylat-Gruppen kommt.

Die gegenüber wässrigen Flüssigkeiten abdichtende Polymerfolie kann beispielsweise mittels Extrusion, Heißpressen oder Lösungsgießen hergestellt werden.

Im Verfahrensschritt C) kann die Vliesschicht durch Elektrospinnen oder Schmelzspinnen erzeugt werden. Bevorzugt wird in einem Verfahrensschritt C) die Vliesschicht durch

Elektrospinnen oder Schmelzspinnen bereitgestellt und dabei auch auf der abdichtenden Polymerfolie abgeschieden.

Gemäß einer bevorzugten Ausführungsform des erfindungsgemäßen Verfahrens wird im Verfahrensschritt C) die Vliesschicht, umfassend Polyurethan-Fasern mit einem Durchmesser von 0,7 µm bis 2,7 µm als Vlies bei einem elektrischen Potenzial von 15 bis 39 kV durch Elektrospinnen auf der abdichtenden Polymerfolie abgeschieden. Die Konzentration des Polymers beträgt von 20 bis 60 Gewichtsprozent, bevorzugt von 25 bis 50 Gewichtsprozent, bezogen auf das Gesamtgewicht der Spinnlösung.

Alternativ kann die Vliesschicht in einem Verfahrensschritt C) als Schicht separat von der elastomeren und biokompatiblen Stützschicht und der abdichtenden Polymerfolie bereitgestellt werden. Anschließend kann die Vliesschicht dann im Verfahrensschritt C) beispielsweise mittels Kleben auf der abdichtenden Polymerfolie angeordnet werden.

Im Folgenden wird die Erfindung anhand von Figuren und Ausführungsbeispielen näher erläutert. Es zeigen:
Fig. 1a) bis 1d) verschiedene Ausführungsformen von erfindungsgemäßen Stützschichten mit einer unterschiedlichen Anzahl von Verbindungsstegen zwischen zwei beabstandeten Flächenbereichen,
Fig. 2a) und 2b) unterschiedliche Ausführungsformen von erfindungsgemäßen Stützschichten, auf die abdichtende Polymerfolien laminiert wurden,
Fig. 3a) und 3b) unterschiedliche Ausführungsformen von röhrenförmigen Hohlkörpern, die durch röhrenförmig gerollte Stützschichten gebildet werden, auf die abdichtende Polymerfolien laminiert wurden,
Fig. 4a) und 4b) unterschiedliche Ausführungsformen von röhrenförmigen Hohlkörpern, bei denen die Stützschichten auf andere Art und Weise gerollt werden, wie bei den röhrenförmigen Hohlkörpern der Figuren 3a) und 3b),
Fig. 5a) und 5b) beispielhafte Stützschichten einer anderen erfindungsgemäßen Ausführungsform, die aus einer sich wiederholenden Anordnung von Hohlräumen als Wiederholeinheiten als sogenannte Wabenstruktur aufgebaut sind,
Fig. 6a) bis 6c) rasterelektronenmikroskopische Aufnahmen von verschiedenen elektrogesponnenen Vliesschichten aus Polyurethan,
Fig. 7a) und 7b) ein Spannungs-Dehnungs-Diagramm einer Ausführungsform eines erfindungsgemäßen Gewebeersatzes,
Fig. 8 ein Spannungs-Dehnungs-Diagramm einer Ausführungsform eines erfindungsgemäßen Gewebeersatzes im Vergleich zu einem Spannungs-Dehnungs-Diagramm eines humanen Perikards,
Fig. 9 Fluoreszenz-Aufnahmen nach einer Lebend/Tot-Färbung von auf der Vliesschicht wachsenden menschlichen Fibroblasten und menschlichen Epithelzellen,
Fig. 10 ein schematisches Verfahren zur Herstellung des Gewebeersatzes durch Elektrospinnen, und
Fig. 11 eine schematische Explosionszeichnung eines erfindungsgemäßen biokompatiblen Gewebeersatzes mit einer elastomeren und biokompatiblen Stützschicht, einer abdichtenden Folie und einer Vliesschicht auf der abdichtenden Folie.

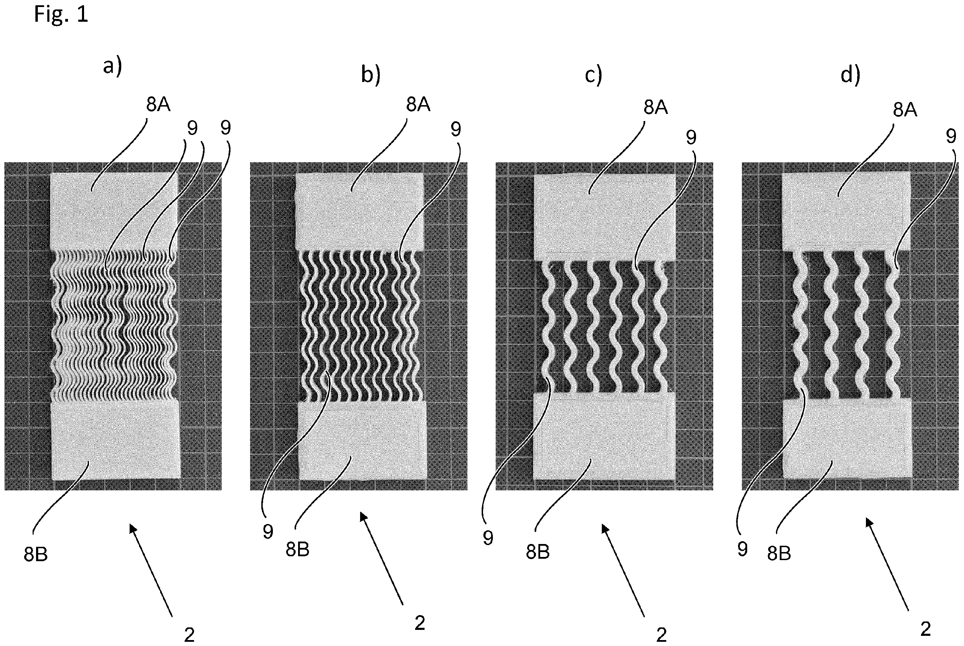

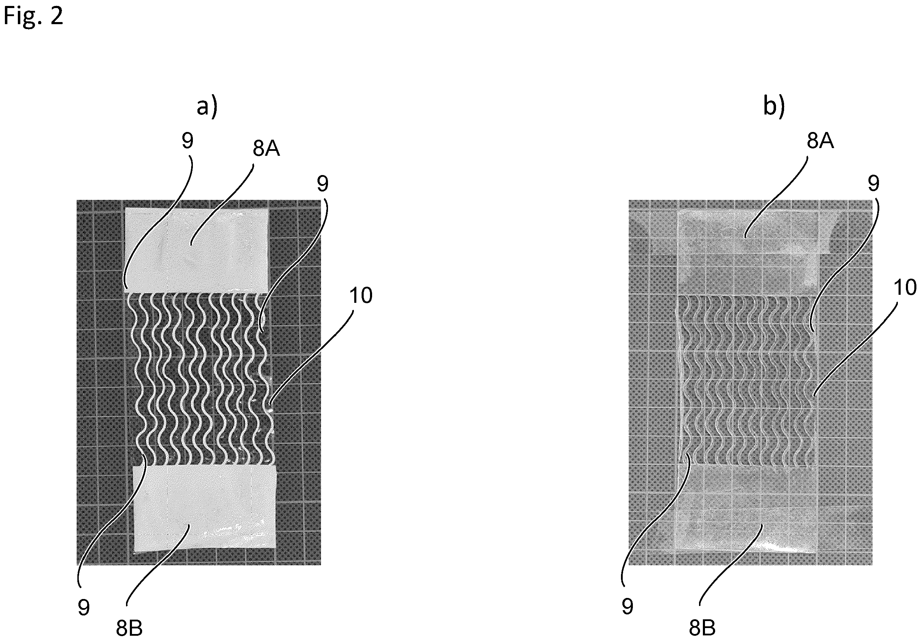

Die Figuren 1a) bis 1d) zeigen unterschiedliche Ausführungsformen von Stützschichten 2, die zwei voneinander beabstandete Flächenbereiche, einen ersten Flächenbereich 8A und einen zweiten Flächenbereich 8B aufweisen, die durch eine unterschiedliche Anzahl von sich schlängelnden Verbindungsstegen 9 miteinander verbunden sind. Dabei nimmt von der Figur 1a) bis zur Figur 1d) die Anzahl der sich schlängelnden Verbindungsstege zwischen dem ersten Flächenbereich 8A und im zweiten Flächenbereich 8B ab. Beispielsweise sind vier schlängelnde, den ersten Flächenbereich mit dem zweiten Flächenbereich verbindende Verbindungsstege in der Stützschicht der Figur 1d) vorhanden. In der Stützschicht der Figur 1c) sind bereits sechs verbindende Verbindungsstege zu sehen, während bei der Stützschicht der Figur 2b) zwölf sich schlängelnde Verbindungsstege vorhanden sind. Die Zugkraft der Stützschichten steigt dabei linear mit der Anzahl der sich schlängelnden Verbindungsstege zwischen dem ersten Flächenbereich und dem zweiten Flächenbereich bei gleichbleibender Breite der Verbindungsstege an. Für die aufzubringende Zugkraft bei einer Stützschicht ist die Gesamtbreite der sich schlängelnden Verbindungsstege ausschlaggebend. Diese Stützschichten können beispielsweise mittels 3-D-Druck hergestellt werden. Aus Gründen der Übersichtlichkeit ist die Vliesschicht und die abdichtende Polymerfolie des biokompatiblen Gewebeersatzes in diesen Figuren nicht gezeigt.

Fig. 2a) und 2b) zeigen eine Anordnung aus einer Stützschicht mit jeweils einem ersten Flächenbereich 8A und einem zweiten Flächenbereich 8B und die die Flächenbereiche verbindenden Verbindungsstege 9, auf die eine abdichtende, transparente Polymerfolie 10 laminiert ist. Bei der in der Figur 2a) gezeichneten Anordnung wurde die Stützschicht mittels 3D-Druck hergestellt, während bei der in der Figur 2b) gezeigten Anordnung die Stützschicht mittels Laserschnitt strukturiert wurde. Aus Gründen der Übersichtlichkeit ist die Vliesschicht in diesen Figuren nicht gezeigt. Derartige Anordnungen können beispielsweise nach Anordnung einer zusätzlichen Vliesschicht auf der abdichtenden Polymerfolie als biokompatibler Gewebeersatz für das Perikard verwendet werden und beispielsweise als Beutel ausgeformt sein.

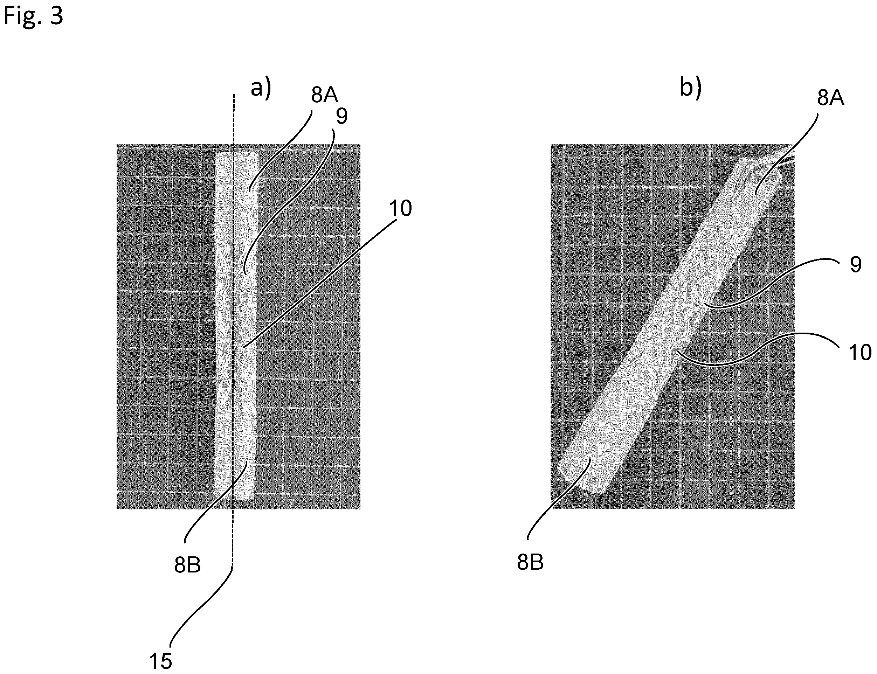

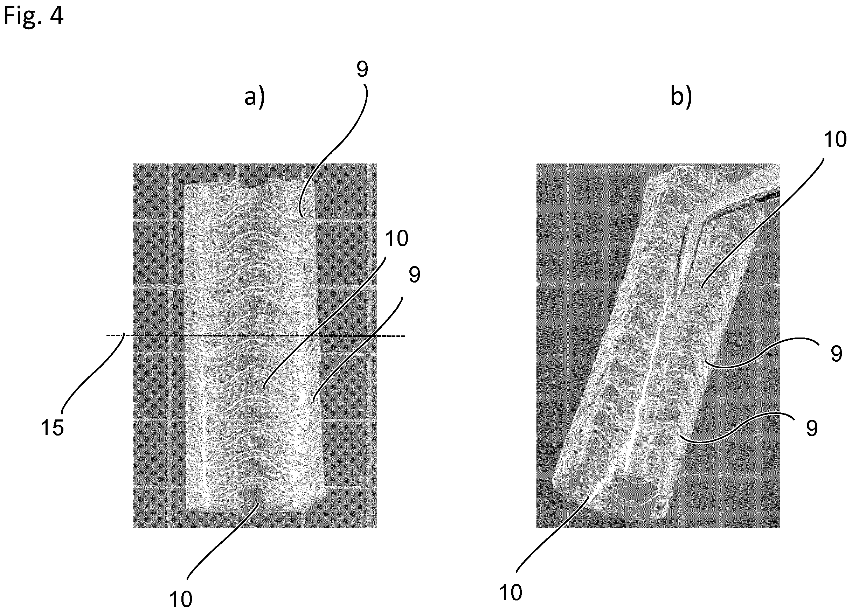

Die Fig. 3a) und 3b) zeigen eine weitere Ausführungsform einer erfindungsgemäßen Anordnung aus einer Stützschicht und einer abdichtenden Polymerfolie 10, die röhrenförmig ausgeformt ist. Die Stützschicht weist dabei eine Längsachse 15 auf, die vom ersten Flächenbereich über die sich schlängelnden Verbindungsstege zum zweiten Flächenbereich verläuft. Der röhrenförmige Körper der Anordnung der Figuren 4a) und 4b) wird dadurch ausgeformt, dass die Stützschicht mit der abdichtenden Polymerfolie um diese Längsachse herum gewickelt wird. Derartige Anordnungen können zusammen mit einer auf der abdichtenden Polymerfolie 10 angeordneten Vliesschicht (Vliesschicht aus Gründen der Übersichtlichkeit nicht in den Figuren 3a) oder 3b) gezeigt) als Gewebeersatz zum Beispiel für Gefäße verwendet werden.

Die Fig. 4a) und 4b) zeigen weitere Ausführungsformen einer erfindungsgemäßen Anordnung aus einer Stützschicht und einer abdichtenden Polymerfolie 10, die röhrenförmig ausgeformt sind. Im Gegensatz zu den röhrenförmigen Anordnungen der Figuren 3a) und 3b) sind diese röhrenförmigen Anordnungen senkrecht zu der Längsachse 15 gewickelt und der erste Flächenbereich und der zweite Flächenbereich wurden abgetrennt.

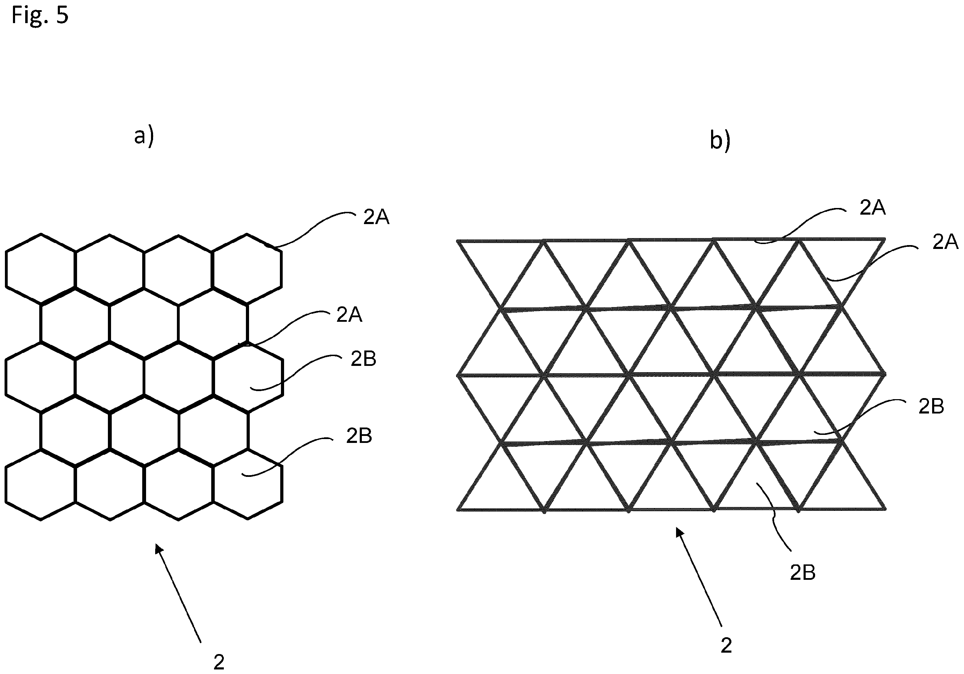

Die Figuren 5a) und 5b) zeigen verschiedene andere Ausführungsformen einer Stützschicht 2, die aus einer sich wiederholenden Anordnung von Wiederholeinheiten aufgebaut sind. In der Figur 5a) ist eine Wabenstruktur gezeigt, bei der die Stützschicht sich aus hexagonalen Hohlräumen 2B aufbaut, die durch Trennwände 2A voneinander abgetrennt sind. Eine derartige Stützschicht ist besonders geeignet eine für das Perikard geeignete nicht-lineare Zugfestigkeit des Gewebeersatzes zur Verfügung zu stellen. In der Figur 5b) ist eine Wiederholstruktur gezeigt, bei der die Stützschicht 2 sich aus wiederholenden trigonalen Hohlräumen 2B aufbaut, die durch Trennwände 2A voneinander getrennt sind.

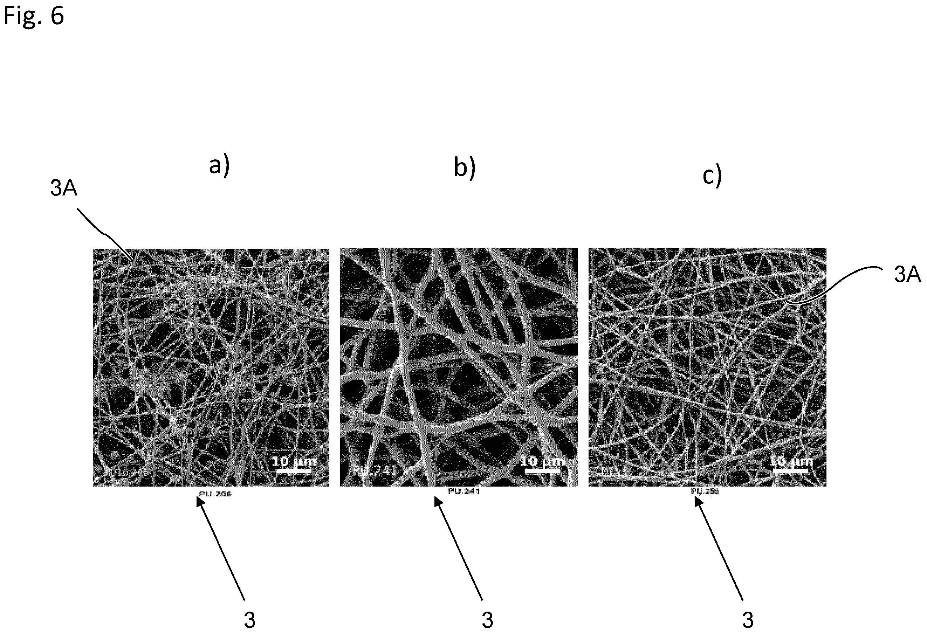

Die Figuren 6a) bis 6c) zeigen rasterelektronenmikroskopische Aufnahmen von verschiedenen Vliesschichten 3, die unterschiedliche gesponnene Fasern 3A mit unterschiedlichen durchschnittlichen Durchmessern aufweisen.

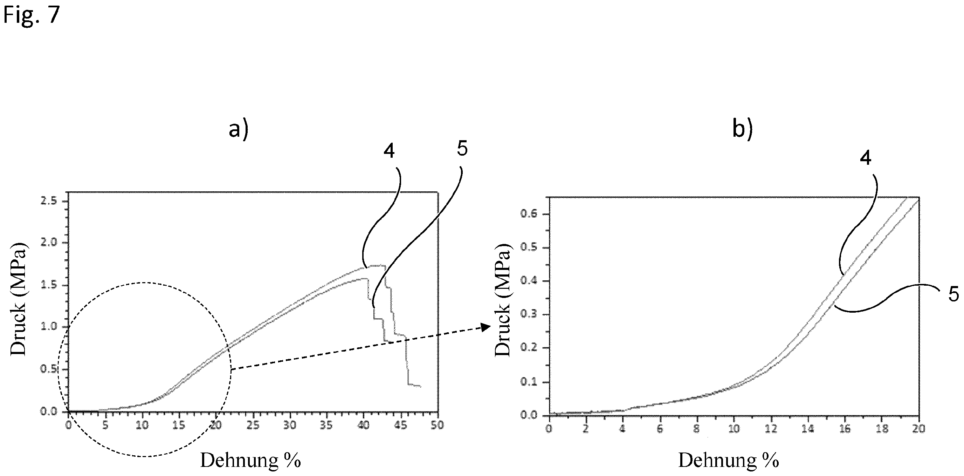

Fig. 7a) und 7b) zeigen ein Spannungs-Dehnungs-Diagramm einer Ausführungsform eines erfindungsgemäßen Gewebeersatzes mit zwei unterschiedlichen Zugversuchen. Die mit 4 und 5 bezeichneten Graphen zeigen die unterschiedlichen Zugversuche, die mit dem gleichen Gewebe durchgeführt wurden. Die Figur 7b) zeigt dabei vergrößert den Bereich der Dehnung von 0 % bis 20 % der Figur 7a). Die Proben für die Zugversuche waren 11 cm × 5 cm große Folien und wurden gemäß ISO 527 gemessen. Die elastomere und biokompatible Stützschicht, und die abdichtende Polymerfolie wurden alle aus Polyurethan gefertigt. Die Vliesschicht wurde aus mit Kollagen funktionalisiertem Polyurethan gefertigt. Die Vliesschicht wurde mit dem Lösungsmittel THF auf die abdichtende Polymerfolie geklebt. Die Stützschicht hatte eine Dicke zwischen 100 µm und 500 µm mit einer Dichte von 1,20 g/cm³, einer Shore-Härte von 85A, einer Zugfestigkeit von 62,1 MPa, einer Bruchdehnung von 400%, einem Zugmodul bei 100% Dehnung von 6,0 MPa und einem Biegemodus von 24,1 MPa. Die sich schlängelnden Verbindungsstege haben eine Breite von 400 µm bis 3 mm. Der Abstand zwischen den sich schlängelnden Verbindungsstegen beträgt 1,2 mm bis 9 mm.

Die abdichtende Polymerfolie hat eine Dicke von 100 µm, eine Dichte von 1,19 g/cm³. Eine Shore-Härte von 77A, eine Zugfestigkeit von 55,2 MPa, eine Bruchdehnung von 400%, ein Zugmodul bei 100% Dehnung von 2,8 MPa und einen Biegemodus von 10,3 MPa

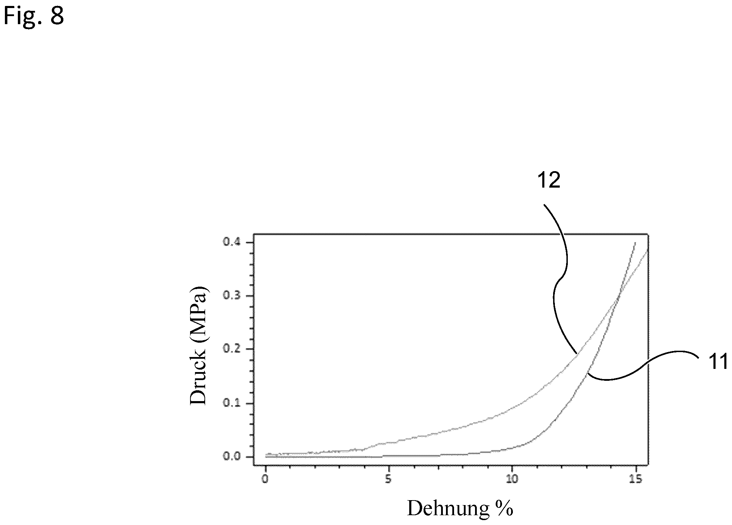

Figur 8 zeigt ein Spannungs-Dehnungs-Diagramm der Zugversuche eines erfindungsgemäßen biokompatiblen Gewebeersatzes (der mit 12 bezeichnete Graph) im Vergleich zu einem analogen Zugversuch mit menschlichem Perikard (der mit 11 bezeichnete Graph). Der Figur 8 lässt sich entnehmen, dass sowohl der biokompatible Gewebeersatz wie auch das zu ersetzende Perikard eine ähnlich verlaufende nichtlineare Dehnung in einem Bereich der Dehnung von 0 % bis 20 % aufweisen. Die Proben der Figur 8 hatten die gleichen Ausmaße wie die Proben der Zugversuche der Figuren 7a) und 7b).

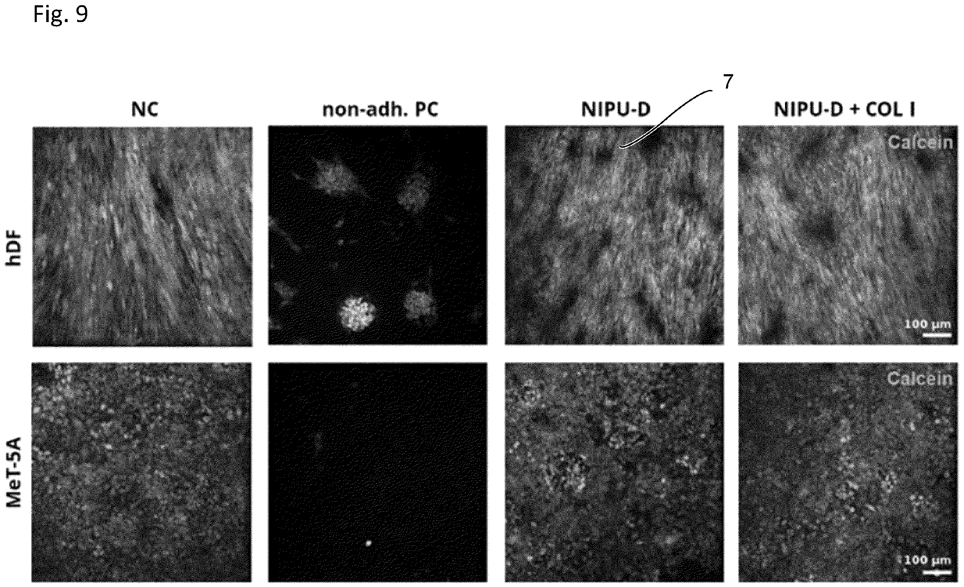

Figur 9 zeigt verschiedene Fluoreszenz-Aufnahmen von humanen Zellen, die auf Vliesschichten kultiviert wurden. Die Fluoreszenz-Aufnahmen zeigen humane Zellen nach Lebend/Tot-Färbung mit dem fluoreszierenden Farbstoff Calcein In lebenden Zellen wird das nicht-fluoreszierende Calcein AM nach der Acetoxymethylester-Hydrolyse durch intrazelluläre Esterasen in grün fluoreszierendes Calcein umgewandelt. Die obere Reihe der Fluoreszenz-Aufnahmen zeigt die Färbung von humanen dermalen Fibroblasten (hDF) auf Glas als Negativkontrolle (NC), die Färbung auf Parafilm als Positivkontrolle (non-adh. PC), die Färbung auf einer Vliesmatte aus Isocyanat-freiem Polyurethan (NIPU-D) und die Färbung auf einer Vliesmatte aus isocyanat-freiem Polyurethan, die mit Kollagen funktionalisiert wurde (NIPU-D + COL I). Die untere Reihe zeigt die entsprechenden Fluoreszenz-Aufnahmen von humanen Epithelzellen (MeT-5A). Deutlich ist zu erkennen, dass analog zur Positivkontrolle lebende humane Fibroblasten und humane Epithelzellen, die auf der PU-Vliesmatte oder der mit Kollagen funktionalisierten PU-Vliesmatte kultiviert wurden, eingefärbt werden können. Sowohl auf der PU-Vliesmatte wie auch auf der mit Kollagen funktionalisierten PU-Vliesmatte konnten kultivierte Zellen nach 24 Stunden Kultivierung positiv mit Calcein angefärbt werden. Nach 7 Tagen Kultivierung auf beiden Vliesmatten konnte eine zusammenhängende Schicht von Zellen positiv angefärbt werden.

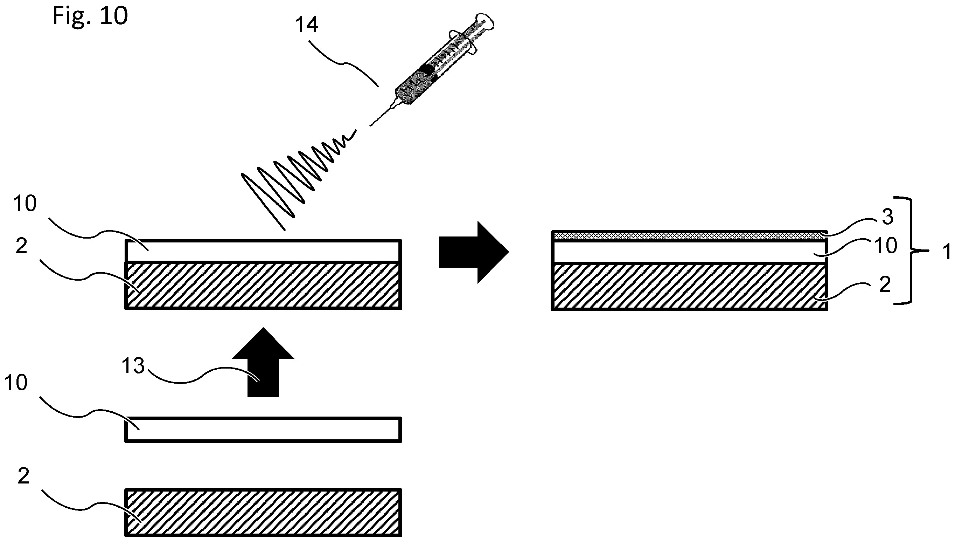

Figur 10 zeigt schematisch ein Verfahren zur Herstellung von erfindungsgemäßen Gewebeersatz. Dabei wird in einem ersten Verfahrensschritt A) eine abdichtende Polymerfolie 10 und eine Stützschicht 2 bereitgestellt. Anschließend kann im Verfahrensschritt B), angedeutet durch den mit 13 bezeichneten Pfeil, beispielsweise durch Laminierung die abdichtende Polymerfolie 10 auf der Stützschicht 2 angeordnet werden. Daran anschließend kann dann in einem Verfahrensschritt C) beispielsweise mittels Elektrospinnen, angedeutet durch die Spritze 14 eine Vliesschicht 3 auf der abdichtenden Polymerfolie 10 erzeugt werden. Alternativ ist es auch möglich, die Vliesschicht 3 zuerst mittels Elektrospinnen herzustellen und dann diese separate Vliesschicht 3 zum Beispiel mittels Kleben auf der abdichtenden Polymerfolie 10 anzuordnen. Nach dem Verfahrensschritt C) ist dann der biokompatible Gewebeersatz 1 bereitgestellt.

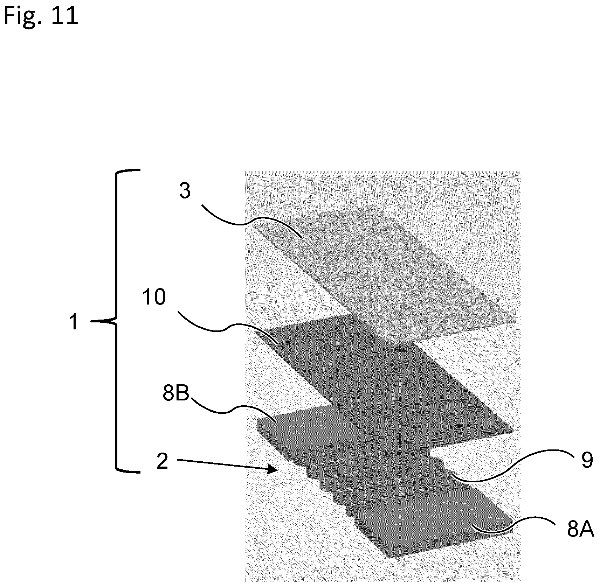

Figur 11 zeigt eine schematische Explosionszeichnung eines erfindungsgemäßen biokompatiblen Gewebeersatzes 1 mit der Stützschicht 2, die einen ersten Flächenbereich 8A und einen zweiten Flächenbereich 8B aufweist, die durch sich schlängelnde Verbindungsstege 9 verbunden sind. Auf dieser Stützschicht 2 ist die, gegenüber wässrigen Flüssigkeiten abdichtende Polymerfolie 10 angeordnet und wiederum auf der abdichtenden Polymerfolie 10 die Vliesschicht 3.

Die Erfindung ist nicht durch die Beschreibung anhand der Ausführungsbeispiele beschränkt. Vielmehr umfasst die Erfindung jedes neue Merkmal sowie jede Kombination von Merkmalen, was insbesondere jede Kombination von Merkmalen in den Patentansprüchen beinhaltet, auch wenn dieses Merkmal oder diese Kombination selbst nicht explizit in den Patentansprüchen oder Ausführungsbeispielen angegeben ist.

## Patentansprüche

1. Biokompatibler Gewebeersatz, umfassend
- eine elastomere, und biokompatible Stützschicht,
- eine Vliesschicht, wobei die Vliesschicht gesponnene, biokompatible Polymerfasern umfasst, und
- eine gegenüber wässrigen Flüssigkeiten abdichtende Polymerfolie,
- wobei die abdichtende Polymerfolie zwischen der Vliesschicht und der Stützschicht angeordnet ist.

2. Gewebeersatz nach dem vorhergehenden Anspruch, wobei die Stützschicht als Körper, bevorzugt als Hohlkörper, weiter bevorzugt als Beutel oder als röhrenförmiger Körper ausgeformt ist.

3. Gewebeersatz nach einem der vorhergehenden Ansprüche, wobei die Stützschicht einen ersten Flächenbereich und einen vom ersten Flächenbereich beabstandeten zweiten Flächenbereich umfasst, wobei zumindest ein, sich schlängelnder Verbindungssteg den ersten Flächenbereich mit dem zweiten Flächenbereich verbindet, bevorzugt wobei eine Vielzahl von nebeneinander sich schlängelnden Verbindungsstegenden den ersten Flächenbereich mit dem zweiten Flächenbereich verbinden.

4. Gewebeersatz nach einem der vorhergehenden Ansprüche, wobei die Stützschicht im Spannungs-Dehnungs-Diagramm eine nicht-lineare Dehnung in einem Bereich einer Dehnung zwischen 0 % und 20 % aufweist, wobei das Spannungs-Dehnungs-Diagramm gemäß der Norm DIN EN ISO 527 bestimmt wird, bevorzugt wobei die Stützschicht eine Zugfestigkeit von 0,003 N/mm² bis 0,09 N/mm² bei einer Dehnung zwischen 0% und 10% und eine Zugfestigkeit von 0,09 N/mm² bis 0,69 N/mm² bei einer Dehnung zwischen 10% und 20% aufweist.

5. Gewebeersatz nach einem der vorhergehenden Ansprüche, wobei die Stützschicht ein elastomeres Polymer umfasst, wobei das elastomere Polymer der Stützschicht ausgewählt ist aus einer Gruppe bestehend aus: Polyurethanelastomeren, Silikonelastomeren, Polyisopren, Olefinelastomeren, Ethylen-Propylen-Dien-Kautschuken, Ethylen-Propylen-Kautschuke, Styrol-Blockcopolymere und Polyetherblock-Amiden, Acrylat-Elastomere und deren Copolymere, bevorzugt Copolymere mit Methyacrylat oder Methylmethacrylat, weiter bevorzugt Isocyanat-freie Polyurethanelastomere, weiter bevorzugt Polyurethanmethacrylat.

6. Gewebeersatz nach einem der vorhergehenden Ansprüche, wobei die Dicke der Stützschicht von 50 µm bis 300 µm, bevorzugt von 80 µm bis 150 µm beträgt.

7. Gewebeersatz nach einem der vorhergehenden Ansprüche, wobei die Polymere der gesponnenen Polymerfasern der Vliesschicht ausgewählt sind aus: Polyurethan, Polymilchsäure (PLA), Polycaprolacton, Poly(lactid-co-glycolid), Polyethylenoxid, Polyvinylalkohol und Strukturproteinen oder Co-Polymeren davon, bevorzugt isocyanat-freies Polyurethan, Elastin, Kollagen.

8. Gewebeersatz nach einem der vorhergehenden Ansprüche, wobei die gesponnenen Polymerfasern der Vliesschicht einen durchschnittlichen Durchmesser zwischen 50 nm und 6000 nm aufweisen, bevorzugt wobei die gesponnen Polymerfasern einen durchschnittlichen Durchmesser von 50 nm bis 3000 nm aufweisen.

9. Gewebeersatz nach einem der vorhergehenden Ansprüche, wobei die Vliesschicht Polyurethan mit einem Molekulargewicht von wenigstens 14000 g/mol, bevorzugt Polyurethan mit einem Molekulargewicht von wenigstens 18000 g/mol, weiter bevorzugt Polyurethan mit einem Molekulargewicht von 18000 g/mol bis 60000 g/mol umfasst.

10. Gewebeersatz nach einem der vorhergehenden Ansprüche, wobei die gesponnenen, biokompatiblen Polymerfasern der Vliesschicht ineinander verschlungene Wirrfasern sind, bevorzugt, wobei die Polymerfasern vereinzelte, nicht miteinander verschmolzene Polymerfasern sind.

11. Gewebeersatz nach einem der vorhergehenden Ansprüche, wobei die Vliesschicht direkt auf der abdichtenden Polymerfolie angeordnet ist und/oder wobei die abdichtende Polymerfolie direkt auf der Stützschicht angeordnet ist, bevorzugt wobei die Vliesschicht direkt auf der abdichtenden Polymerfolie abgeschieden ist.

12. Gewebeersatz nach einem der vorhergehenden Ansprüche, bestehend aus der Stützschicht, der abdichtenden Polymerfolie und der Vliesschicht.

13. Verwendung des Gewebeersatzes nach einem der vorhergehenden Ansprüche als Implantat oder Wundauflage.

14. Verwendung des Gewebeersatzes nach dem vorhergehenden Anspruch, als Implantat für das Perikard, Gefäß-Implantat oder als Herzklappe.

15. Verfahren zur Herstellung eines Gewebeersatzes nach einem der vorhergehenden Ansprüche 1 bis 12 mit den Verfahrensschritten:
A) Bereitstellen der elastomeren und biokompatiblen Stützschicht, und der abdichtenden Polymerfolie,
B) Anordnen der der abdichtenden Polymerfolie auf der Stützschicht, und
C) Erzeugen oder Anordnen der Vliesschicht auf der abdichtenden Polymerfolie.
